# EUROPÄISCHE PATENTSCHRIFT

(11) **EP 1 143 236 B1**
(45) Veröffentlichungstag und Bekanntmachung des Hinweises auf die Patenterteilung: **31.10.2007**
(21) Anmeldenummer: 01100854.7
(22) Anmeldetag: 16.01.2001
(51) Int. Cl.: G01N 21/89, G01N 33/36, D06H 3/08, G01N 21/892

(54) **Verfahren und Vorrichtung zur optischen Detektion von Verunreinigungen, insbesondere Fremdfasern, in längsbewegtem Garn**
Process and device for optical detection of impurities, especially fibres, in advancing yarn
Procédé et dispositif de détection optique d'impurtés, en particulier de fibres, dans des fils à coudre défilants

(30) Priorität: 26.02.2000 DE 10009131
(43) Veröffentlichungstag der Anmeldung: 10.10.2001
(73) Patentinhaber: Oerlikon Textile GmbH & Co. KG, 41069 Mönchengladbach (DE)
(72) Erfinder: Henze, Herbert, 41063 Möchengladbach (DE); Birlem, Olav, 41366 Schwalmtal (DE)

(56) Entgegenhaltungen:
- EP-A- 0 652 432
- EP-A- 0 761 585
- EP-B- 0 399 945
- WO-A-93/19359
- WO-A-95/29396
- WO-A-98/33061
- DE-U- 29 719 245
- US-A- 4 739 176
- US-A- 5 499 794

## Beschreibung

Die Erfindung betrifft ein Verfahren zur optischen Detektion von Verunreinigungen nach dem Oberbegriff des Anspruchs 1 und eine Vorrichtung zur optischen Detektion von Verunreinigungen nach dem Oberbegriff des Anspruchs 7.

Die Erkennung von Verunreinigungen ist bei der Garnherstellung von großer Bedeutung. Verunreinigungen, insbesondere durch Fremdfasern, können sich auf das textile Endprodukt außerordentlich nachteilig auswirken.

Aus der US 4,739,176 A ist es bekannt, Garn auf Fremdstoffe zu überwachen, indem das Garn vor einem Hintergrund, der das gleiche Reflexionsvermögen wie das Garn aufweist, geführt wird. Damit soll erreicht werden, daß die Menge des reflektierten Lichtes, das vom Garn und vom Hintergrund reflektiert wird, in der Summe im wesentlichen unabhängig vom Durchmesser des Garns ist und eine Veränderung im reflektierten Licht keine Veränderung des Garndurchmessers anzeigt, sondern eine Verunreinigung, z. B. infolge von Fremdfasern. Die Reflexion von Hintergrund und Garn muss dabei sehr genau aufeinander abgestimmt sein, da andernfalls Dick- oder Dünnstellen im Garn Veränderungen der Summe des reflektierten Lichtes verursachen und die Ergebnisse der Fremdstoffkennung verfälschen. Der Hintergrund wird durch die Oberflächen eines semi-opaken Keramik-Einsatzes gebildet. Der Einsatz muss gleiche Farbe oder Helligkeit bzw. gleiches Reflexionsvermögen wie das Garn aufweisen. Die einmal vorgenommene Abstimmung gilt nur für ein bestimmtes Garn und wird für die gesamte Partie beibehalten. In der Vorrichtung zum Überwachen von Garn auf Fremdstoffe werden vier Lampen eingesetzt. Die Lampen illuminieren den semi-opaken Keramik-Einsatz. Die Lampen emittieren Licht, das reflektiert und vom Sensor detektiert wird. Das vom Garn allein reflektierte Licht ist nicht vom Einfluss des Garndurchmessers befreit, sondern abhängig vom Garndurchmesser. Erst die Summe des vom Garn und vom Hintergrund reflektierten Lichts ist im wesentlichen unabhängig vom Durchmesser des Garns. Die Abstimmung bzw. der Austausch ist aufwändig und muss zudem bei jeder Veränderung des Fasermaterials, wie z. B. nach einem Partiewechsel, neu vorgenommen werden. Bei einem Wechsel des Fasermaterials muss der Einsatz manuell ausgetauscht werden. Neben dem Erfordernis relativ hohen Arbeitsaufwandes zur Anpassung des Hintergrundes an das zu prüfende Garn je nach Art oder Typ des Garns reagiert das Verfahren sehr empfindlich auf Verschmutzung und Alterung des Hintergrundes. Verschmutzung tritt unvermeidlich häufig in Textilbetrieben und gerade an Spulstellen auf. Die Veränderung des Hintergrunds durch Verschmutzung und Alterung führt zu einer Ergebnisverfälschung bei der Fremdstoffkennung und erfordert ein Auswechseln des als Hintergrund dienenden Einsatzes.

Auch aus der EP 0 553 445 ist es bekannt, das Garn vor einem Hintergrund zu überwachen, der das gleiche Reflexionsvermögen wie das Garn aufweist. Dabei treten ebenfalls die oben angeführten Nachteile auf.

Die WO 93 193 59 A1 beschreibt ein Verfahren und eine Vorrichtung zur Detektion von Verunreinigungen in einem textilen Prüfgut. Das Prüfgut wird an mindestens zwei Stellen beleuchtet, und es wird durch Empfänger die Reflexion des Prüfguts und zusätzlich zu dieser der Durchmesser des Prüfguts oder dessen Änderung gemessen. Die so erhaltenen Messsignale werden miteinander verknüpft. Das aus diesem Prozess resultierende Signal wird auf Abweichungen von einem vorgegebenen Wert untersucht. Wird eine Abweichung festgestellt, dann liegt eine Verunreinigung des Prüfguts vor. Vorzugsweise wird aus dem Reflexions- und aus dem Durchmessersignal ein Quotient abgeleitet und fortlaufend auf Abweichungen von einem Mittelwert überprüft. Das Verknüpfen der Messsignale erfordert hohen Rechenaufwand. Bei einer derartigen Signalverknüpfung werden die Signale üblicherweise als digitale Signale verarbeitet. Bei der Umsetzung von analogen Signalen in digitale Signale kommt es durch die Umwandlung in diskrete Werte zu einem Diskretisierungsfehler, der bei einer Fremdfasererkennung zur Verfälschung der Messergebnisse führen kann. Bei Veränderungen des Reflexionssignals können die Signalanteile, die durchmesserabhängig sind, das 10fache oder sogar das 100fache der fremdfaserabhängigen Signalanteile betragen. Kleine Fehler bei der Verknüpfung der Signale und der Eliminierung des durchmesserabhängigen Signalanteils können zu erheblicher Verfälschung des geringen Anteils des Fremdfasersignals am Gesamtsignal und damit zu entsprechender Verfälschung des Ergebnisses der Fremdfasererkennung führen.

Die US 5,499,794 A offenbart ein Verfahren und eine Vorrichtung zur Detektion von Fremdstoffen in einem textilen Prüfgut wie z.B. Garn mittels optischer Sensoren. Es sollen Fremdstoffe detektiert werden, die heller oder dunkler sind als das betrachtete Garn. Dabei wird das Garn zur Detektion von dunkleren Fremdstoffen als das Garn vor hellem und zur Detektion von helleren Fremdstoffen vor dunklem Hintergrund auf einen Sensor abgebildet. Je nach Art des Fremdstoffes, der detektiert werden soll, wird wahlweise für den Hintergrund, vor dem gemessen wird, der Zustand "beleuchtet" (hell) oder der Zustand "nicht beleuchtet" (dunkel) vorgesehen. Das Signal des Sensors wird jeweils mit einem einstellbaren Grenzwert verglichen. Nachteilig dabei ist das Erfordernis des Anpassens des Hintergrundes bei Garnwechsel und die Beschränkung auf eine gewählte vorbestimmte Fremdstoffart. In Helligkeit bzw. Farbe andersartige Fremdstoffe werden mit der jeweils in Verbindung mit einem Grenzwert gewählten Einstellung nicht erfasst und verbleiben nachteilig im Garn. Die Vorrichtung der US 5,499,794 A arbeitet mit einem Zeilensensor. Zeilensensoren weisen eine Vielzahl von einzelnen Sensorelementen auf, die jeweils ein Signal abgeben, so dass bei jeder Messung an einem Punkt des Garns eine Vielzahl von Signalen erzeugt wird. Die Fremdstoffe seien daher gut detektierbar. Gegenüber einem einfachen optischen Sensor, der nur aus einem Element besteht, tritt beim Einsatz von Zeilensensoren ein erhöhter Raumbedarf sowie gesteigerter Kosten- und Auswertungsaufwand auf. Besonders der Kostenaufwand für einen Zeilensensor an jeder Arbeitsstelle stellt bei der Vielzahl von Arbeitsstellen einer Spul- oder Spinnspulmaschine einen entscheidenden Nachteil dar.

Die EP 0 553 446 und die EP 0 572 592 offenbaren Verfahren und Vorrichtungen zur Erkennung von Fremdfasern, bei denen von einer Beleuchtungseinrichtung Licht auf das bewegte Garn geworfen wird, wo es reflektiert und transmittiert wird. Das reflektierte Licht und das transmittierte Licht werden jeweils in ein elektrisches Signal gewandelt. Diese beiden elektrischen Signale werden miteinander verknüpft und dadurch ein weiteres elektrisches Signal gewonnen, bei dem die Fremdfasern angezeigt und die sonstigen Garnfehler wie Dick- oder Dünnstellen unterdrückt werden. Bei einer derartigen Signalverknüpfung werden die Signale üblicherweise als digitale Signale wie o.a. verarbeitet, wodurch die oben erläuterten Nachteile auftreten. Aus der gattungsbildenden EP 0 572 592 ist zudem eine Einrichtung bekannt, mit der durch Verschmutzungen verursachte Änderungen des Lichtes der Lichtquelle kompensiert werden, indem die Intensität der Lichtquelle mittels dieser Einrichtung konstant gehalten wird. Damit sollen sich langfristig beziehungsweise langzeitlich entwickelnde Veränderungen des Lichtes, wie sie durch die Verschmutzung entstehen können, vermieden werden.

Aufgabe der Erfindung ist es, die Nachteile bei der bekannten Erkennung von Fremdfasern zu vermindern oder zu beseitigen und die Detektion von Verunreinigungen in längsbewegtem Garn zu verbessern.

Diese Aufgabe wird durch ein Verfahren mit den Merkmalen des Anspruchs 1 oder durch eine Vorrichtung mit den Merkmalen Merkmalen des Anspruchs 7 gelöst.

Weitere vorteilhafte Ausgestaltungen der Erfindung sind Gegenstand der Unteransprüche.

Durch die erfindungsgemäße Ausbildung, bei der jeweils nach der Messung durch den Sensor die Lichtquelle in ihrer Intensität abhängig vom ersten elektrischen Signal so eingestellt wird, daß bei durchmesserabhängig eingestellter Intensität des aufgestrahlten Lichts das vom Garn remittierte Licht vom Einfluß der Größe des Garndurchmessers befreit ist, kann ein zweites elektrisches Signal gebildet werden, das bereits unmittelbar der Erkennung von Verunreinigungen dienen kann. Eine Abstimmung des Reflexionsvermögens des Meßhintergrundes mit dem Reflexionsvermögen des Garns ist dazu nicht mehr erforderlich. Weil durchmesserabhängige Signalanteile erfindungsgemäß erst gar nicht mehr in das zweite Signal eingehen und Veränderungen des zweiten Signals direkt Verunreinigungen anzeigen, können Rechenschritte zur Verknüpfung des zweiten Signals mit einem anderen beziehungsweise einem weitgehend durchmesserabhängigen Signal zur Eliminierung der Durchmesserabhängigkeit und zur Erkennung des Fremdfasersignalanteils entfallen. Die durch derartige Rechenschritte verursachten Diskretisierungsfehler sowie die Summierung dieser Fehler werden vermieden.

Ein weiteres Vermeiden von Diskretisierungsfehlern erfolgt dadurch, daß das erste elektrische Signal als analoges Signal erzeugt wird. Eine mögliche unerwünschte Beeinflussung von Meßwerten wird auf einfache Weise dadurch vermieden, daß das erste und das zweite elektrische Signal mit vorgegebener Taktfrequenz erfaßt werden oder daß die Lichtquelle mit vorgegebener Taktfrequenz gepulst wird.

Bei der erfindungsgemäßen Steuerung der Intensität des emittierten Lichtes zur Gewinnung des zweiten elektrischen Signals liegt ein ganz anderer Wirkungsmechanismus vor als bei der aus der EP 0 572 592 bekannten Einrichtung zum Regeln der Leistung der Lichtquelle. Während mit der bekannten Einrichtung Veränderungen kompensiert und die Lichtintensität immer konstant gehalten werden soll, wird mit der erfindungsgemäßen Steuerung die Intensität des emittierten Lichtes zur Bildung des zweiten elektrischen Signals im Gegensatz zur bekannten Einrichtung eben gerade nicht konstant gehalten, sondern abhängig vom ersten elektrischen Signal und damit durchmesserabhängig eingestellt. Das bedeutet, daß die Lichtintensität der Lichtquelle für die Bildung des zweiten elektrischen Signals bei einer auftretenden Veränderung des ersten elektrischen Signals erfindungsgemäß verändert wird, wobei bei größerem Garndurchmesser die Lichtintensität entsprechend geringer und bei kleinerem Garndurchmesser die Lichtintensität entsprechend höher eingestellt wird.

Der Aufwand zum Einstellen der Intensität der Lichtquelle abhängig vom ersten elektrischen Signal läßt sich dadurch herabsetzen, daß für die erste Messung zur Bildung des ersten Signals jeweils ein konstanter Wert der Lichtintensität der Lichtquelle eingestellt wird. Dadurch erfolgt die durchmesserabhängige erste Messung zur Bildung des ersten Signals stets unter gleichen Voraussetzungen, und das erste Signal kann unmittelbar als Maß für den momentanen Durchmesser des Garns dienen. Das erste elektrische Signal kann nicht nur zur Steuerung der Intensität einer Lichtquelle eingesetzt werden, sondern zusätzlich für die Überwachung von Dick- und Dünnstellen im Garn ausgewertet werden.

In einer bevorzugten Ausbildung des Verfahrens wird das zweite elektrische Signal mit einer Zeitverzögerung gegenüber dem ersten elektrischen Signal an einer Meßstelle gewonnen, die in bezug auf die Meßstelle des ersten elektrischen Signals stromab in Richtung des Fadenlaufs liegt, und die Zeitverzögerung wird in Abhängigkeit von der Garngeschwindigkeit so gesteuert, daß beide Signale an derselben Stelle des Garns beziehungsweise im selben Garnabschnitt gewonnen werden. Damit ist eine außerordentlich hohe Meßgenauigkeit und Meßsicherheit gewährleistet.

Eine Einrichtung zur Kompensation der durch Alterung der Lichtquellen oder durch Verschmutzungen auftretenden Veränderungen in der Lichtintensität kann zusätzlich eingesetzt werden.

Das erfindungsgemäß gewonnene zweite elektrische Signal führt zu einer erhöhten Meßempfindlichkeit bei der optischen Detektion von Verunreinigungen. Kritische Bereiche, wie zum Beispiel die Unterscheidung von dunkelbraun und schwarz oder die Unterscheidung von wollweiß und reinweiß, lassen sich mit Hilfe des Erfindungsgegenstandes problemlos beherrschen. Die Erfindung erlaubt eine deutliche Verbesserung bei der Detektion von Verunreinigungen, insbesondere von Fremdfasern, in längsbewegtem Garn.

Weitere vorteilhafte Ausgestaltungen der Erfindung sind den in den Figuren dargestellten Ausführungsbeispielen zu entnehmen.

Es zeigen:
- Fig. 1: eine schematische Darstellung einer Spulstelle,
- Fig. 2: eine schematische Darstellung einer erfindungsgemäßen Detektionseinrichtung,
- Fig. 3 und 4: schematische Darstellungen weiterer Ausführungsbeispiele erfindungsgemäßer Detektionseinrichtungen.

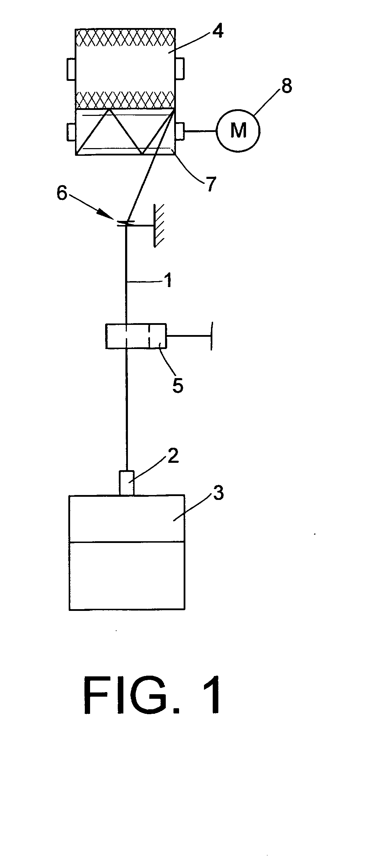

In der in Fig. 1 dargestellten Spinnstelle wird das Garn 1 durch das Abzugsröhrchen 2 aus der Spinnbox 3 abgezogen und auf die Kreuzspule 4 aufgewickelt. Das Garn 1 durchläuft zwischen Abzugsröhrchen 2 und Kreuzspule 4 einen Reiniger 5 und eine Führungsöse 6. Die Antriebstrommel 7 treibt die Kreuzspule 4 während des Aufwickelvorganges mittels Reibschluß an. Ein Motor 8 erteilt der Antriebstrommel 7 die Drehbewegung. Der Reiniger 5 dient der Qualitätsüberwachung des laufenden Garns 1. Der Reiniger 5 umfaßt einen integrierten Meßkopf. Der Reiniger 5 ist mit weiteren Einrichtungen zur Steuerung, Datenspeicherung oder -auswertung und der Ansteuerung von weiteren Elementen der Spinnstelle beziehungsweise der Spinnmaschine verbunden.

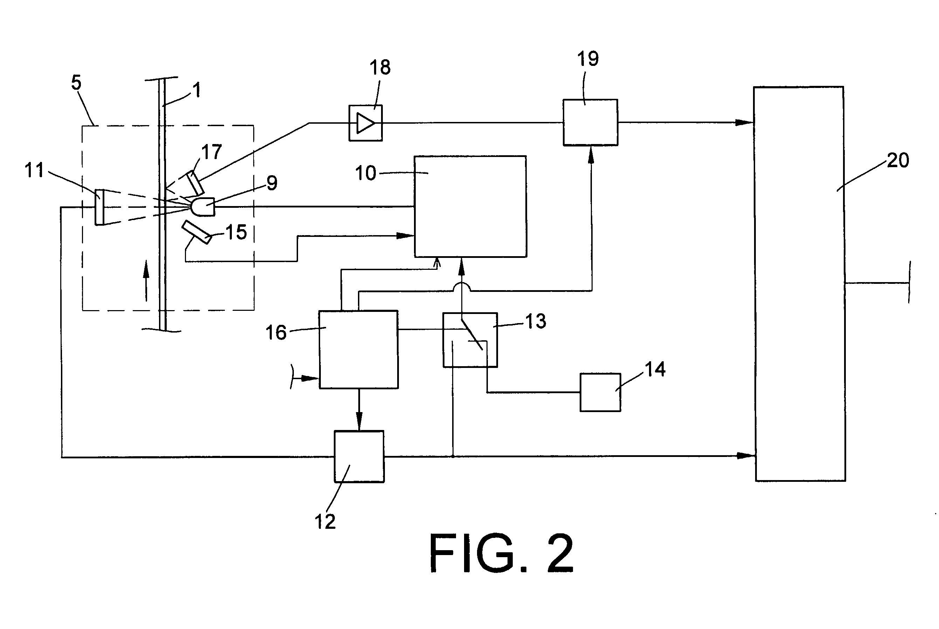

Die in Fig. 2 vereinfachter Form dargestellte Detektionsvorrichtung weist die Lichtquelle 9 und die Steuereinrichtung 10 auf. Die Lichtquelle 9 emittiert Licht in Richtung des Garns 1 und bildet das Garn 1 in an sich bekannter Weise auf dem Sensor 11 ab. Der Sensor 11 führt eine erste Messung durch und bildet ein für den momentanen Garndurchmesser repräsentatives Signal, das in ein analoges elektrisches Signal gewandelt wird. Dieses Signal wird dem Speicher 12 einer Speichereinrichtung zugeführt und dort abgespeichert. Die Steuereinrichtung 10 wird in dieser ersten Phase des Meßvorgangs über den Umschalter 13 mit einem Signal mit einem konstant gehaltenen Wert aus dem Datenspeicher 14 beaufschlagt. Diese Schaltstellung wird in Fig. 2 gezeigt. Das Signal aus dem Datenspeicher 14 dient als Regelsignal für die Einstellung der Intensität des Lichtes, das von der Lichtquelle 9 in dieser ersten Meßphase emittiert wird. Von der Lichtquelle 9 emittiertes Licht wird vom Sensor 15 erfaßt, wobei dieses erfaßte Licht nicht dem Einfluß des Garns 1 unterliegt. Der Sensor 15 ist Teil einer an sich bekannten Regelung, durch die der Einfluß von Verschmutzung oder Alterung der Lichtquelle eliminiert wird und die ein Konstanthalten der Intensität des von der Lichtquelle 9 in der ersten Meßphase emittierten Lichtes bewirkt.

Die Dauer dieser ersten Meßphase wird von einer Steuervorrichtung 16 gesteuert. Die Steuervorrichtung 16 ist mit dem Speicher 12 und der Steuereinrichtung 10 sowie dem Umschalter 13 und weiteren Elementen der Spinnstelle verbunden. Die Steuervorrichtung 16 beendet die erste Meßphase durch Betätigen des Umschalters 13. Durch das Betätigen des Umschalters 13 wird auch die Steuereinrichtung 10 mit dem Steuersignal aus dem Speicher 12 beaufschlagt und die zweite Meßphase beginnt. Dieses analoge elektrische Signal aus dem Speicher 12 repräsentiert den momentanen Durchmesser des Garns 1 in der ersten Meßphase. Die Steuereinrichtung 10 stellt abhängig von diesem Signal die Intensität der Lichtquelle 9 nunmehr so ein, daß das vom Garn 1 remittierte Licht vom Einfluß der Größe des Garndurchmessers befreit ist. Vom Garn 1 remittiertes Licht wird vom Sensor 17 erfaßt und ein zweites elektrisches Signal gebildet, das über einen Verstärker 18 einem Speicher 19 zugeleitet wird. Das zweite elektrische Signal wird von dem Speicher 19 der Auswerteeinrichtung 20 übermittelt und unmittelbar zur Erkennung von Verunreinigungen ausgewertet. Tritt eine Veränderung im remittierten Licht und damit im zweiten Signal auf, repräsentiert die Veränderung eine Verunreinigung.

Vom Speicher 12 wird das erste elektrische Signal ebenfalls der Auswerteeinrichtung 20 übermittelt und dort zur Qualitätsüberwachung des längsbewegten Garns 1 in an sich bekannter Weise ausgewertet.

Nach Vorliegen des zweiten elektrischen Signals beendet die Steuervorrichtung 16 die zweite Meßphase durch Betätigen des Umschalters 13 und beaufschlagt die Steuereinrichtung 10 wieder mit dem Signal aus dem Datenspeicher 14. Der zweiphasige Meßzyklus beginnt damit von vorn. Die Taktfrequenz der Meßzyklen wird von der Steuervorrichtung 16 gesteuert. Die Taktfrequenz der Messungen liegt im Kilohertz-Bereich. Dabei folgen die Messungen so schnell aufeinander, daß dies einer kontinuierlichen Messung gleichzusetzen ist. Die Lichtquelle 9 wird bei Betätigung des Umschalters 13 ausgeschaltet und unmittelbar nach Beendigung des Umschaltvorganges wieder eingeschaltet.

Die beim Ausführungsbeispiel der Fig. 2 innerhalb des gestrichelt angedeuteten Bereiches angeordneten Elemente befinden sich in einem gemeinsamen Gehäuse im Meßkopf des Reinigers 5.

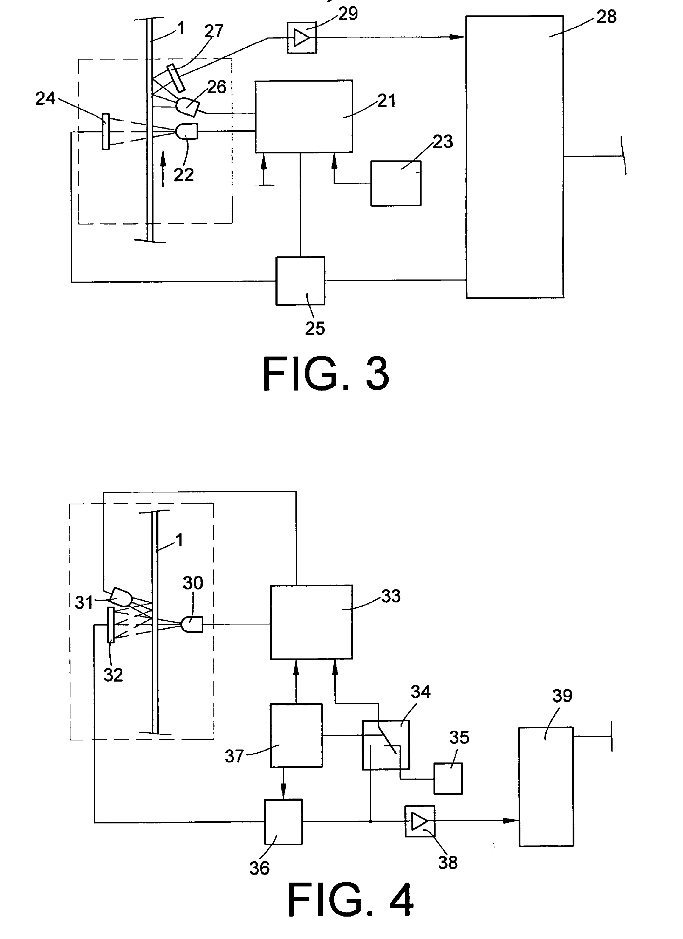

In Fig. 3 ist ein alternatives Ausführungsbeispiel der Vorrichtung dargestellt. Die Lichtquelle 22 wird von der Steuereinrichtung 21 gesteuert. Die Steuereinrichtung 21 ist mit einem Datenspeicher 23 verbunden und wird von dort mit einem Steuersignal zur Einstellung der Intensität des von der Lichtquelle 22 emittierten Lichtes beaufschlagt. Dieses Steuersignal wird konstant gehalten. In einer weiteren alternativen Ausführungsform kann ein Regelkreis vorhanden sein, der in bekannter Weise Alterung und Verschmutzung der Lichtquelle 22 kompensiert.

Das von der Lichtquelle 22 emittierte Licht bildet das Garn 1 auf dem Sensor 24 ab. Der Sensor 24 bildet ein Signal, das den momentanen Durchmesser des Garns repräsentiert und das als erstes analoges elektrisches Signal des Speichers 25 zugeführt wird. Mit diesem Signal aus dem Speicher 25 wird die Steuereinrichtung 21 zur Steuerung der Lichtquelle 26 beaufschlagt. Mittels des Signals aus dem Speicher 25 wird die Lichtquelle 26 in ihrer Intensität so eingestellt, daß das vom Garn remittierte Licht vom Einfluß der Größe des Garndurchmessers befreit ist. Die Messung des remittierten Lichtes erfolgt mittels Sensor 27.

Der Sensor 27 bildet aus dieser zweiten Messung ein zweites elektrisches Signal, das der Auswerteeinrichtung 28 über einen Verstärker 29 zugeführt wird. Dabei erfolgt die Messung des zweiten elektrischen Signals mit einer Zeitverzögerung gegenüber der Messung des ersten elektrischen Signals. Die Zeitverzögerung ist so gesteuert, daß beide Messungen an zwei beabstandeten Meßstellen, aber an derselben Stelle des Garns 1 beziehungsweise im selben Garnabschnitt erfolgen. Um die Zeitverzögerung auf die Garngeschwindigkeit abstimmen zu können, ist die Steuereinrichtung mit Einrichtungen zur Detektion der Garngeschwindigkeit verbunden und erhält von diesen aus Vereinfachungsgründen nicht dargestellten Einrichtungen Signale, die die momentane Garngeschwindigkeit repräsentieren.

Das zweite elektrische Signal wird in der Auswerteeinrichtung 28 unmittelbar zur Erkennung von Verunreinigungen ausgewertet.

Die Meß- und Steuervorgänge laufen kontinuierlich ab. Alternativ kann eine Taktung von Meßphasen innerhalb der Meßzyklen erfolgen.

Neben der Erkennung von Verunreinigungen erfolgt zusätzlich eine Qualitätsüberwachung durch Auswertung des von dem Speicher 25 an die Auswerteeinrichtung 28 übermittelten jeweils ersten elektrischen Signals.

In einem weiteren alternativen Ausführungsbeispiel der Vorrichtung, das in Fig. 4 dargestellt ist, wird das von zwei Lichtquellen 30, 31 emittierte Licht von einem einzigen Sensor 32 gemessen. In einer ersten Meßphase wird die Steuereinrichtung 33 über einen Umschalter 34 vom Datenspeicher 35 mit einem konstant gehaltenen Steuersignal beaufschlagt. Diese Schaltstellung wird in Fig. 4 gezeigt. Die Lichtquelle 30 wird in der ersten Meßphase von der Steuereinrichtung 33 eingeschaltet und in ihrer Intensität auf einen Wert abhängig vom Steuersignal aus dem Datenspeicher 35 so eingestellt, daß die Intensität in jeder ersten Meßphase gleich ist. Das von der Lichtquelle 30 emittierte Licht bildet das Garn 1 auf dem Sensor 32 ab. Der Sensor 32 bildet aus dem erfaßten Licht ein erstes Signal, das den Durchmesser des Garns 1 repräsentiert. Dieses Signal wird als analoges elektrisches Signal dem Speicher 36 zugeführt und dort abgespeichert. Die Steuereinrichtung beendet die erste Meßphase dadurch, daß die Lichtquelle 30 ausgeschaltet wird. Die Taktfrequenz des Ein- und Ausschaltens wird der Steuereinrichtung 33 durch die mit ihr verbundene Steuervorrichtung 37 vorgegeben.

Die Steuervorrichtung 37 betätigt nunmehr den Umschalter 34. Nach Betätigen des Umschalters 34 wird die Steuereinrichtung 33 mit dem ersten elektrischen Signal aus dem Speicher 36 beaufschlagt. Die Lichtquelle 31 wird von der Steuereinrichtung 33 eingeschaltet und in dieser zweiten Meßphase in ihrer Intensität abhängig vom ersten elektrischen Signal so eingestellt, daß das vom Garn 1 remittierte Licht vom Einfluß der Größe des Garndurchmessers befreit ist.

Vom Garn 1 in der zweiten Meßphase remittiertes Licht wird vom Sensor 32 gemessen und ein zweites Signal gebildet, das als elektrisches Signal dem Speicher 36 zugeführt wird. Sowohl das erste elektrische Signal wie auch das zweite elektrische Signal werden vom Speicher 36 über einen Verstärker 38 an die Auswerteeinrichtung 39 übermittelt. Die Lichtquelle 31 wird anschließend von der Steuereinrichtung 33 wieder ausgeschaltet. Die Steuervorrichtung 37 betätigt den Umschalter 34 und beendet dadurch die zweite Meßphase und damit den gesamten ersten Meßzyklus.

Nach Betätigen des Umschalters 34 wird die Steuereinrichtung 33 wieder mit dem Signal aus dem Datenspeicher 35 beaufschlagt, und der zweite Meßzyklus beginnt. Die zweite Messung wird zur ersten Messung derart zeitverzögert durchgeführt, daß die zweite Messung an derselben Stelle des Garns erfolgt wie die erste Messung. Die Zeitverzögerung wird auf die momentane Garngeschwindigkeit in an sich bekannter Weise abgestimmt.

Alternativ zu den Ausführungsbeispielen können Steuer- und Speicherelemente in einem einzigen Mikroprozessor oder in anderer Aufteilung innerhalb oder außerhalb des Meßkopfes angeordnet sein.

## Patentansprüche

1. Verfahren zur optischen Detektion von Verunreinigungen, insbesondere von Fremdfasern, in längsbewegtem Garn, wobei Licht in Richtung des Garns emittiert wird und die Intensität des transmittierten Lichtes gemessen und in ein erstes elektrisches Signal gewandelt wird, dessen Größe vom momentanen Durchmesser des Garns abhängig ist, und wobei vom Garn remittiertes Licht ebenfalls gemessen und ein zweites elektrisches Signal gebildet wird,
**dadurch gekennzeichnet,**
**daß** jeweils nach der Bildung des ersten elektrischen Signals die Intensität des emittierten Lichtes, abhängig vom ersten elektrischen Signal, so eingestellt wird, daß bei der dann durchmesserabhängig eingestellten Intensität des aufgestrahlten Lichts das vom Garn (1) remittierte Licht vom Einfluß der Größe des Garndurchmessers befreit ist und daß das zweite elektrische Signal unmittelbar der Erkennung von Verunreinigungen dient.

2. Verfahren nach Anspruch 1, **dadurch gekennzeichnet, daß** als erstes elektrisches Signal ein analoges Signal erzeugt wird.

3. Verfahren nach Anspruch 1 oder 2, **dadurch gekennzeichnet, daß** das erste und das zweite elektrische Signal mit vorgegebener Taktfrequenz erfaßt werden.

4. Verfahren nach einem der vorhergehenden Ansprüche, **dadurch gekennzeichnet, daß** das emittierte Licht mit vorgegebener Taktfrequenz gepulst wird.

5. Verfahren nach einem der vorhergehenden Ansprüche, **dadurch gekennzeichnet, daß** für die erste Messung zur Bildung des ersten Signals jeweils ein konstanter Wert der Lichtintensität eingestellt wird.

6. Verfahren nach einem der vorhergehenden Ansprüche, **dadurch gekennzeichnet, daß** das zweite elektrische Signal mit einer Zeitverzögerung gegenüber dem ersten elektrischen Signal an einer Meßstelle gewonnen wird, die in bezug auf die Meßstelle des ersten elektrischen Signals stromab in Richtung des Fadenlaufs liegt, und **daß** die Zeitverzögerung in Abhängigkeit von der Garngeschwindigkeit so gesteuert ist, **daß** beide Signale an derselben Stelle des Garns (1) gewonnen werden.

7. Vorrichtung zur optischen Erfassung von Verunreinigungen, insbesondere von Fremdfasern, in längsbewegtem Garn mit einer Beleuchtungseinrichtung, von der Licht in Richtung des Garns emittiert wird, mit einer Sensoreinrichtung, die so ausgebildet ist, daß sie sowohl transmittiertes Licht erfaßt und in ein elektrisches Signal wandelt, dessen Größe vom momentanen Durchmesser des Garns abhängig ist, als auch vom Garn remittiertes Licht mißt und daraus ein zweites elektrisches Signal bildet, sowie mit einer Steuereinrichtung zur Steuerung der Beleuchtungseinrichtung,
**dadurch gekennzeichnet,**
**daß** die Steuereinrichtung (10, 21, 33) so ausgebildet ist, daß sie jeweils mittels des in einer ersten Meßphase bei einer ersten Messung von einem Sensor (11,24,32) gebildeten Signals eine Lichtquelle (9, 26, 31) der Beleuchtungseinrichtung in ihrer Intensität abhängig vom ersten elektrischen Signal so einstellt, daß bei der dann durchmesserabhängig eingestellten Intensität des aufgestrahlten Lichts das vom Garn (1) remittierte Licht vom Einfluß der Größe des Garndurchmessers befreit ist, und daß eine Auswerteeinrichtung (20, 28, 39) vorhanden ist, mit der das bei einer zweiten Messung gebildete zweite elektrische Signal unmittelbar zur Erkennung von Verunreinigungen auswertbar ist.

8. Vorrichtung nach Anspruch 7, **dadurch gekennzeichnet, daß** die Beleuchtungseinrichtung zwei Lichtquellen (22, 26; 30, 31) aufweist, wovon eine erste Lichtquelle (22, 30) nur Licht für die erste Messung und die zweite Lichtquelle (26, 31) nur Licht für die zweite Messung emittiert.

9. Vorrichtung nach einem der Ansprüche 7 oder 8, **dadurch gekennzeichnet, daß** die Sensoreinrichtung zwei Sensoren (11, 17; 24, 27) aufweist, wovon ein erster Sensor (11, 24) jeweils nur Licht bei der ersten Messung und ein zweiter Sensor (17, 27) jeweils nur reflektiertes Licht bei der zweiten Messung erfaßt.

10. Vorrichtung nach Anspruch 9, **dadurch gekennzeichnet, daß** der zweite Sensor (17, 27), der vom Garn (1) remittiertes Licht mißt, so angeordnet ist, **daß** er vom ersten Sensor (11, 24), auf dem das Garn (1) nur vom Licht der ersten Lichtquelle (22,30) abgebildet ist, beabstandet ist und in bezug auf den ersten Sensor (11, 24) stromab in Richtung des Fadenlaufs liegt.

11. Vorrichtung nach einem der Ansprüche 7 bis 10, **dadurch gekennzeichnet, daß** eine Steuervorrichtung (16, 37) vorhanden ist, die so ausgebildet ist, **daß** die Weiterverarbeitung der Signale aus der ersten und der zweiten Messung jeweils abtastfrequenzabhängig gesteuert ist .

12. Vorrichtung nach einem der Ansprüche 7 bis 11, **dadurch gekennzeichnet, daß** eine Speichereinrichtung mit mindestens einem Speicher (12, 25, 36) zur Speicherung des jeweils ersten Signals vorhanden ist und **daß** die Speichereinrichtung mit einer Steuervorrichtung (16) verbunden ist.

13. Vorrichtung nach einem der Ansprüche 7 oder 8, **dadurch gekennzeichnet, daß** ein Umschalter (13, 34) vorhanden ist, der so ausgebildet und gesteuert ist, **daß** zur Steuerung der Intensität der Lichtquelle (9, 30, 31) die Steuereinrichtung (10, 33) entweder mit einem Steuersignal von einem Datenspeicher (14, 35) oder mit einem Steuersignal von einem das erste Signal abspeichernden Speicher (12, 36) beaufschlagbar ist.

## Claims

1. Method for the optical detection of impurities, in particular foreign fibres, in a longitudinally travelling yarn, wherein light is emitted in the direction of the yarn and the intensity of the transmitted light is measured and converted into a first electrical signal, the size of which is dependent on the current diameter of the yarn, and wherein light reflected by the yarn is also measured and a second electrical signal is formed, **characterised in that** after the formation of the first electrical signal the intensity of the emitted light, dependent on the first electrical signal, is adjusted so that, at the intensity of light dependent on diameter, the light reflected by the yarn (1) is not influenced by the size of the yarn diameter, and **in that** the second electrical signal is used directly for identifying impurities.

2. Method according to claim 1, **characterised in that** an analogue signal is generated as the first electrical signal.

3. Method according to claim 1 or 2, **characterised in that** the first and second electrical signals are detected at a predetermined dock frequency.

4. Method according to one of the preceding claims, **characterised in that** the emitted light is pulsed at a predetermined dock frequency.

5. Method according to one of the preceding claims, **characterised in that** for the first measurement to produce the respective first signal a constant value is set for the light intensity.

6. Method according to one of the preceding claims, **characterised in that** the second electrical signal is obtained after a time delay relative to the first electrical signal at a measuring point located downstream in the travelling direction of the yarn relative to the measuring point of the first electrical signal, and **in that** the time delay is controlled as a function of the yarn velocity so that both signals are obtained at the same point of the yarn (1).

7. Device for the optical detection of impurities, in particular foreign fibres, in a longitudinally travelling yarn with a lighting device from which light is emitted in the direction of the yarn, with a sensor device which is designed so that it both detects transmitted light and converts it into an electrical signal, the size of which is a function of the current diameter of the yarn, and also measures light reflected by the yarn and from this forms a second electrical signal and with a control device for controlling the lighting device, **characterised in that** the control device (10, 21, 33) is designed so that by means of the signal formed in a first measuring phase in a first measurement of a sensor (11, 24, 32) it adjusts a light source (9, 26, 31) of the lighting device in its intensity as a function of the first electrical signal, so that, at the intensity of light adjusted as a function of the diameter, the light reflected by the yarn (1) is not influenced by the size of the yarn diameter, and **in that** an evaluation device (20, 28, 39) is provided, by means of which the second electrical signal formed by a second measurement can be evaluated directly to identify impurities.

8. Device according to claim 7, **characterised in that** the lighting device comprises two light sources (22, 26; 30, 31), including a first light source (22, 30) which only emits light for the first measurement and a second light source (26, 31) which only emits light for the second measurement.

9. Device according to one of claims 7 or 8, **characterised in that** the sensor device comprises two sensors (11, 17; 24, 27) including a first sensor (11, 24) which only detects light in the first measurement and a second sensor (17, 27) which only detects reflected light in the second measurement.

10. Device according to claim 9, **characterised in that** the second sensor (17, 27), which measures light reflected by the yarn (1), is arranged so that it is spaced apart from the first sensor (11, 24), at which the yarn (1) is displayed only by the light of the first light source (22, 30), and said second sensor is positioned downstream of the direction of the yarn in relation to the first sensor (11, 24).

11. Device according to one of claims 7 to 10, **characterised in that** a control device (16, 37) is provided, which is designed so that the processing of the signals from the first and second measurement is controlled as a function of the frequency of scanning.

12. Device according to one of claims 7 to 11, **characterised in that** a memory device with at least one memory (12, 25, 36) is provided for storing the respective first signal, and **in that** the memory device is connected to a control device (16).

13. Device according to one of claims 7 or 8, **characterised in that** a changeover switch (13, 34) is provided, which is designed and controlled so that to control the intensity of the light source (9, 30, 31) the control device (10, 33) can either be charged by a control signal from a data memory (14, 35) or by a control signal from a memory (12, 36) storing the first signal.

## Revendications

1. Procédé de détection optique d'impuretés, en particulier de fibres étrangères, dans du fil à coudre défilant longitudinalement, de la lumière étant émise en direction du fil et l'intensité de la lumière transmise étant mesurée et convertie en un premier signal électrique, dont la grandeur dépend du diamètre momentané du fil, et de la lumière renvoyée par le fil étant également mesurée et un second signal électrique étant formé, **caractérisé en ce que**, chaque fois après la formation du premier signal électrique, l'intensité de la lumière émise est réglée en fonction du premier signal électrique, de telle sorte que la lumière renvoyée par le fil (1) soit libérée de l'influence de la grandeur du diamètre du fil en présence de l'intensité de la lumière transmise alors réglée en fonction du diamètre et que le second signal électrique serve directement à la reconnaissance d'impuretés.

2. Procédé selon la revendication 1, **caractérisé en ce qu'**un signal analogique est généré en tant que premier signal électrique.

3. Procédé selon la revendication 1 ou 2, **caractérisé en ce que** le premier et le second signal électrique sont détectés suivant une fréquence de cycles prédéfinie.

4. Procédé selon l'une quelconque des revendications précédentes, **caractérisé en ce que** la lumière émise est pulsée suivant une fréquence de cycles prédéfinie.

5. Procédé selon l'une quelconque des revendications précédentes, **caractérisé en ce qu'**une valeur constante de l'intensité lumineuse est réglée chaque fois pour la première mesure destinée à la formation du premier signal.

6. Procédé selon l'une quelconque des revendications précédentes, **caractérisé en ce que** le second signal électrique est obtenu avec un retard de temps par rapport au premier signal électrique, à un point de mesure qui se trouve en aval, dans le sens de défilement du fil, du point de mesure correspondant au premier signal électrique et **en ce que** le retard de temps est commandé en fonction de la vitesse de défilement du fil, de telle sorte que les deux signaux soient obtenus au même endroit du fil (1).

7. Dispositif de détection optique d'impuretés, en particulier de fibres étrangères, dans du fil à coudre défilant longitudinalement, comportant un dispositif d'éclairage à partir duquel de la lumière est émise en direction du fil, un dispositif détecteur, lequel est conformé de telle sorte que non seulement il détecte de la lumière transmise et la convertisse en un signal électrique dont la grandeur dépend du diamètre momentané du fil, mais également mesure de la lumière renvoyée par le fil et forme à partir de celle-ci un second signal électrique, ainsi qu'un dispositif de commande destiné à commander le dispositif d'éclairage, **caractérisé en ce que** le dispositif de commande (10, 21, 33) est conformé de telle sorte qu'il règle l'intensité d'une source lumineuse (9, 26, 31) du dispositif d'éclairage en fonction du premier signal électrique chaque fois au moyen du signal formé par un détecteur (11, 24, 32) lors d'une première mesure effectuée au cours d'une première phase de mesure, de façon à ce que la lumière renvoyée par le fil (1) soit libérée de l'influence de la grandeur du diamètre du fil en présence de l'intensité de la lumière transmise alors réglée en fonction du diamètre, et **en ce qu'**il présente un dispositif d'évaluation (20, 28, 39) au moyen duquel le second signal électrique formé lors d'une seconde mesure peut être évalué directement en vue de la reconnaissance d'impuretés.

8. Dispositif selon la revendication 7, **caractérisé en ce que** le dispositif d'éclairage présente deux sources lumineuses (22, 26; 30, 31), dont une première source lumineuse (22, 30) émet seulement de la lumière pour la première mesure et la seconde source lumineuse (26, 31) seulement de la lumière pour la seconde mesure.

9. Dispositif selon l'une des revendications 7 ou 8, **caractérisé en ce que** le dispositif détecteur présente deux détecteurs (11, 17; 24, 27), dont un premier détecteur (11, 24) détecte chaque fois seulement de la lumière lors de la première mesure et un second détecteur (17, 27) chaque fois seulement de la lumière réfléchie lors la seconde mesure.

10. Dispositif selon la revendication 9, **caractérisé en ce que** le second détecteur (17, 27), lequel mesure de la lumière renvoyée par le fil (1), est disposé de telle sorte qu'il soit distant du premier détecteur (11, 24), sur lequel le fil (1) est représenté seulement par de la lumière de la première source lumineuse (22, 30), et se trouve en aval, dans le sens de défilement du fil, du premier détecteur (11, 24).

11. Dispositif selon l'une quelconque des revendications 7 à 10, **caractérisé en ce qu'**il présente un dispositif de commande (16, 37), lequel est conformé de telle sorte que le traitement ultérieur des signaux provenant de la première et de la seconde mesure soit commandé chaque fois en fonction d'une fréquence de cycles.

12. Dispositif selon l'une quelconque des revendications 7 à 11, **caractérisé en ce qu'**il présente un dispositif de mémoire comportant au moins une mémoire (12, 25, 36) destinée à mémoriser chaque fois le premier signal et **en ce que** le dispositif de mémoire est relié à un dispositif de commande (16).

13. Dispositif selon l'une des revendications 7 ou 8, **caractérisé en ce qu'**il présente un commutateur inverseur (13, 14), lequel est conformé et commandé de telle sorte que le dispositif de commande (10, 33) puisse être alimenté, en vue de la commande de l'intensité de la source lumineuse (9, 30, 31), soit par un signal de commande d'une mémoire de données (14, 35), soit par un signal de commande d'une mémoire (12, 36) mémorisant le premier signal.
